# EUROPEAN PATENT SPECIFICATION

(11) **EP 4 126 056 B1**
(45) Date of publication and mention of the grant of the patent: **26.08.2026**
(21) Application number: 21782035.6
(22) Date of filing: 06.04.2021
(51) Int. Cl.: A61K 47/36, A61K 31/465, A61K 9/00, A61K 9/14, A61K 9/70, A61P 25/34, A61K 9/20, A61K 47/02, A61K 47/12, A61K 47/26, A61K 47/38

(54) **NEW COMPOSITIONS FOR ORAL USE**
NEUE ZUSAMMENSETZUNGEN ZUR ORALEN ANWENDUNG
NOUVELLES COMPOSITIONS POUR UTILISATION ORALE

(30) Priority: 03.04.2020 SE 2050380
(43) Date of publication of application: 08.02.2023
(73) Proprietor: md&c Creative Maison SA, 1206 Geneva (CH)
(72) Inventor: BJÖRKHOLM, Johan, 523 45 ULRICEHAMN (SE); BJÖRKHOLM, Lars, 504 56 BORÅS (SE)
(74) Representative: Papula Oy
(86) International application number: PCT/SE2021/050304
(87) International publication number: WO 2021/201765

(56) References cited:
- EP-A2- 1 790 687
- EP-A2- 1 790 687
- WO-A1-2009/068279
- WO-A1-2017/069721
- WO-A1-2017/180707
- WO-A1-2017/180707
- WO-A1-2018/222581
- WO-A1-2018/222581
- WO-A1-2020/014776
- WO-A2-2010/091649
- WO-A2-2010/091649
- CN-A- 104 784 197
- CN-A- 104 784 197
- CN-A- 107 027 949
- CN-A- 107 027 949
- US-A1- 2010 158 988
- US-A1- 2010 158 988
- US-A1- 2015 098 996
- US-A1- 2015 098 996
- US-B1- 6 499 490

## Description

### Technical field

The present invention relates to compositions for use in the oral cavity comprising a biologically active agent, a matrix forming agent comprising cereal β-glucan and a filling agent and methods of producing the compositions.

### Background art

Delivery of pharmaceutically active agents to oral and nasal cavity is generally a desirable administration route to obtain a fast therapeutic onset and to avoid the metabolic activities of the gastrointestinal system and a first by-pass metabolism. Numerous solid dose forms such as lozenges, sublingual tablets, chewing gums, buccal patches or pouches have been developed to obtain compliant dose forms for patients depending on therapies through the oral cavity. Such solid dose forms typically include active agents, fillers, binders, lubricants and other ingredients supporting mucoadhesiveness, palatability, compliance and release of an active agent.

Suppliers and developers of smokeless tobacco and nicotine products have developed numerous products configured to deliver nicotine through the oral or nasal cavity. Tobacco products include for example chewing tobacco, moist smokeless tobacco, snus and dry snuff to be used orally or nasally. Non-tobacco products rely on pure nicotine extracted from tobacco or synthetic nicotine formulated with suitable additives to an oral or nasal dose form as exemplified. For the oral cavity non-tobacco dose forms can for example rely on a fibrous filler material and a matrix forming agent acting as a binder. Nicotine (3-(1-methyl-2-pyrrolidinyl) pyridine is a volatile compound liable to degradation under the influence of heat, oxygen and light. For this reason, it is a technical challenge to find a suitable non-tobacco dose form as a product that counteracts degradation during its manufacturing and provides a suitable storage stability of nicotine while admitting a desirable release rate of nicotine in the oral cavity and yet is compliant to the user. For these reasons, developers of nicotine dose forms search for new agents to replace tobacco, but support and extended shelf life, a desirable release profile and high compliance for the consumer.

WO 2010/011445 discloses a plant fiber product for oral use suitable for delivery of active agents such as nicotine. The incorporation of alginate as matrix former provides the product with desirable release characteristics and a suitable stabilization of liable active agents.

WO 2010/104464 discloses particles of alginate comprising active agent such as nicotine enclosed in pouches for use in the oral cavity.

WO 2015/051308 and US 2015/0098996 disclose tobacco or nicotine lozenges with at least 40% weight of water soluble fibers primarily of maltodextrin and with less than 15% water. However, nothing is disclosed regarding the shelf life of nicotine or the release properties of nicotine in the oral cavity.

EP1622627 describes pharmaceutical compositions comprising cereal β-glucans and a pharmaceutical agent, suggested to be used in the oral cavity for delivering agents such as a local anesthetic. However, no such product is made or practically tested in this document so it cannot be concluded if cereal β-glucans is a suitable excipient for the delivery of a liable active agent to the oral or nasal cavity.

US2010/158988 describes orally consumable dry, dissolvable films or coatings based on cereal β-glucans that can comprise up to 10% (wt) of a filler. The films, however, do not demonstrate any adaptions to be nicotine products suitable for delivery of nicotine to the oral or nasal cavity. EP1790687 describes similar dry films that may comprise nicotine, but do not teach nicotine dose forms of similar compliance for the consumer as traditional tobacco products.

WO2010091649 relates to a tobacco-free nicotine product for use in the oral cavity thereby describing a pH value. However, there is no disclosure of how to affect stability and release of nicotine with purposefully selected supporting agents.

CN10707494 discloses a chewing gum comprising gum matrix, β-glucan, sweetener, and a cellulose lecithin as an active agent.

CN104784197 a composition specific for delivering the active agent epigallocatechin gallate, comprising β-glucan.

US6499490 disclose a tobacco substitute sheet material comprising β-glucan and leaf tobacco extract.

### Description of the invention

It is an object of the present invention to provide compositions suitable to deliver a biologically active agent to the oral cavity that admits stability of active agent throughout manufacturing and storage, while admitting a suitable release profile of said agent.

It is also an object of the present invention to provide compositions that support a controlled release rate of active agent and a, suitable duration of the release rate.

It is also an object of the present invention to provide compositions that promote stabilization of active agents liable to degradation during storage to obtain stable products with long shelf life also in compositions with relatively high water content.

It is also an object of the present invention to provide compositions with high compliance with the mucosa of the oral cavity in order to avoid local irritation and side-effects from repeated or long term exposure.

It is still another object of the invention to provide compositions suitable to deliver nicotine to the oral cavity and thereby satisfy the user expectations of compliance with comparable and conventional tobacco products or pharmaceutical products.

In a general aspect, the invention is directed to a composition for use in the oral cavity comprising a biologically active agent, a matrix forming agent comprising β-glucan and a filling agent. The invention is directed to a composition according to claim 1 and a method of manufacturing a composition according to claim 10.

In this general context of the invention, a matrix forming agent is capable of together with the filling agent provide cohesive, homogenous compositions that encompass the biologically active agent and contribute to exert a stabilizing effect on liable such agents, while contributing to a controllable and a desirable release profile of the active agent when in contact with the oral cavity. The stabilizing effects and the release profile may in aspect be caused by an interaction or synergy with the filling agent, for example between the matrix forming agent and fibers of a filling agent. Accordingly, in the inventive compositions, the interaction between the active agent and the matrix forming agent can be employed to adjust and control the release rate.

The matrix forming agent of the invention is used also to bind the biologically active agent in a controlled manner in the composition. For example, when the active agent is nicotine, the matrix forming agent can be selected so a controlled amount of nicotine is free, unbound nicotine and a controlled amount of nicotine is gradually and controllably released from the matrix forming agent. For example, by increasing the amount of matrix forming agent in the inventive compositions, more nicotine is bound and gradually released. The compositions can accordingly be developed to provide the user with a satisfying initial dose of nicotine administered to the oral or nasal cavity and be provided with a gradual release of nicotine from the composition during a predetermined time period. For a nicotine product, the matrix forming agent can be used to meet different requests of user compliance.

In one aspect, the compositions of the invention comprise less than 50% (wt) of the matrix forming agent, such as less than 40% (wt) or less than 30% (wt) and less than 20%(wt), or from 0.1 to 10% (wt), or from 0.5 to 5% (wt).

In other aspects, the compositions of the invention comprise more than 50% (wt) of the matrix forming agent, such as from 50 to 90% (wt), or from 50 to 70% (wt).

Also, in this general context, the filling agent will principally contribute to bulk and shape of the composition, for example to admit user compliance in different parts of the oral cavity and/or assist with convenient manufacturing, handling and administration of the compositions. The filling agent may also in embodiments positively interact with the matrix forming agents to stabilize the agent(s) and to induce desirable release properties.

The β-glucan of the compositions is obtainable from various sources including cereals and yeasts and comprises at least 30% β(1-3) β(1-4) glucan, preferably comprising from 70 to 99 or almost 100% β(1-3) β(1-4) glucan. It is preferable that the β-glucan is obtained from cereals and more preferably oat. The cereal β-glucan can be purified to a suitably high grade by methods outlined in for example Journal of Food Science, 2017, 82(9) (G Maheshwari et al) and Chemical Engineering and Processing, 2014, 84, page 90-97 (O Benito-Román et al).

In one aspect, the compositions of the invention include a matrix forming agent comprises at least 50% (weight) of β-glucan. The compositions of the invention can comprise a matrix forming agent that further comprises at least one additional pharmaceutically acceptable gum or gel forming polysaccharide of food or pharmaceutical grade, preferably selected from alginate and suitable salts thereof, xanthan, carrageenan, methyl cellulose, cudlan, pullulan, guar gum, gum arabicum and similar polysaccharides, preferably the additional gum is a salt of alginate, more preferably sodium alginate.

The filling agent of the inventive compositions may comprise a fiber material, which can be of natural or a synthetic source. The fiber is preferably derived from plants, algae or fungi and it can be natural or modified with bioprocesses or chemical methods. In preferred aspects, the fiber material is a plant fiber, more preferably the filling agent comprises natural or modified cellulose fibers and most preferably at least one microcrystalline cellulose.

In various embodiments, the plant fibers comprised in filling agent can be derived from one of tea, coffee, tobacco, cocoa, maize, bamboo, oat, barley, rye, sugar beets, herbs, buckwheat, potatoes, tomatoes, aubergines, cauliflower, apples, yerba mate or cellulose fibers various sources and the similar. The plant fibers can be natural or modified with various biological or chemicals methods. The tobacco fibers may be processed according to various conventional technologies for whiteness and/or reduction of nitrosamines.

Suitable microcrystalline celluloses (MCC) for the filling agent can be selected from AVICEL^{®} grades PH-100, PH-102, PH-103, PH-105, PH-112, PH-113, PH-200, PH-300, PH-302, VIVACEL^{®} grades 101, 102, 12, 20; EMOCEL^{®} grades 50M and 90M, HiCel^{®} grades, such as HiCel^{®} 90M and the like, and mixtures thereof. For embodiments of the inventive compositions, wherein a water soluble microcrystalline cellulose is desirable, grades of colloid microcrystalline cellulose are useful, such as various grades of TABULOSE^{®}.

For embodiments of the inventive compositions, wherein a water-soluble microcrystalline cellulose is desirable, suitable grades of colloid microcrystalline cellulose are the grade with Cas No. 51395-75-6, such as various brands of TABULOSE^{®}. A preferred such colloid gelling MCC has the trade name FEIYUN XW591.

In embodiments of the inventive compositions, the filling agent comprises a polyol, preferably a polyol selected from one or more mannitol, xylitol, sorbitol, maltitol and/or isomaltitol, lactitol and erythritol. Suitably, the inventive compositions comprise a plant fiber material and 5 to 70% (wt) of a polyol, preferably the filling agent comprises mannitol and at least one microcrystalline cellulose.

The biologically active agent is nicotine. A further biologically active agent can be a therapeutic or a non-therapeutic substance not generally considered as a pharmaceutical, such as a naturopathic preparation, a stimulant or a nutraceutical. Examples of therapeutic biologically active substances that can be administered alone or in combinations by the inventive compositions include urinary incontinence agents; antihistamines, analgesics, anti-inflammatory agents, antiemetics, anti-epileptics, vasodilators, antitussive agents and expectorants, anti-spasmodics, hormones, diuretics, anti-hypotensives, bronchodilators, anti-inflammatory steroids, antibiotics, sedatives, CNS-active substances, cannabinoids, such as Δ9-tetrahydrocannabinol (THC) or cannabidiol (CBD), decongestants, laxatives and antacids. Generally, the compositions are useful as drug delivery dose forms for patients suffering from complications leading to incapacity of receiving conventional tables for swallowing such as unconsciousness, severe migraine, acute stroke or gastrointestinal obstructions. Examples of suitable non-therapeutic agents are caffeine, alcohol powder, ethanol, vitamin B12, vitamin C, vitamin E, Bioperin^{®}, Coenzyme Q10, selenium, glutathione, alpha liponic acid, folic acid, ginseng, pollen extract, antioxidants, minerals, paracetamol, acetylsalicylic acid, Russian root and rose root, etc.

In embodiments of the invention, the compositions have a pH of at least 6.5, preferably a pH of 8 to 9 and the biologically active agent is nicotine.

In embodiments, the biologically active agent is nicotine. The term nicotine includes synthetic nicotine and nicotine extracts from tobacco plants such as the genus Nicotiana or other plant sources and includes nicotine or a nicotine derivative in any solid or liquid form, e.g., physical form like amorphous, crystalline, polymorphous etc. or chemical form like enantiomers etc as well as any pharmaceutically acceptable salt, complex or solvate thereof. The term nicotine herein also includes nicotine base and/or salts thereof, such as nicotine hydrochloride, nicotine dihydrochloride, nicotine monotartrate, nicotine bitartrate, nicotine sulphate, nicotine zinc chloride (monohydrate) and nicotine salicylate.

Nicotine is typically present in a concentration from about 0.1% (wt) to about 5% (wt), such as, e.g., from about from about 0.1% (wt) to about 4% (wt), from about 0.1% (wt) to about 3% (wt), from about 0.1% (wt) to about 2% (wt), from about 0.1% (wt) to about 1% (wt), from about 0.1% (wt) to about 0.75% (wt), from about 0.2% (wt) to about 0.5% (wt) or from about 0.2% (wt) to about 0.4% (wt), calculated as free base. The nicotine or its salts used with the inventive compositions preferably is of high purity, such as 99.5 % purity.

The optional antioxidant of the inventive compositions is an antioxidant effective at a pH of at least 6.5, such as a pH of 8 to 9, preferably the antioxidant is a complex binding antioxidant, more preferably the antioxidant is selected from at least one of alkali and/or alkaline earth metal salts of ascorbate, calcium citrates, calcium lactates, calcium maleates, calcium tartrates, Ca-diNa-EDTA, calcium phosphates and ammonium citrates, still more preferably the antioxidant is an ascorbate selected from sodium ascorbyl phosphate, potassium ascorbate calcium ascorbate, calcium ascorbyl phosphate, magnesium ascorbate. Most preferably, the antioxidant is calcium ascorbate. This type of antioxidants are generally preferable when the biologically active agent is nicotine. However, other types of active agents may require complementary or different antioxidants or antioxidant systems in order to obtain a suitable storage stability.

The compositions according to the invention may further comprise at least one excipient selected from plasticizers, pH adjusters, preservatives, taste or flavor enhancers, coloring agents and sweeteners.

The plasticizer can be e.g. polyethylene glycols, propylene glycols, glycerol and sorbitol. A preferred plasticizer is sorbitol, optionally together with a part of glycerol.

The pH adjuster is capable of maintaining a pH of at least 6.5 in the compositions and is exemplified by carbonates including monocarbonate, bicarbonate and sesquicarbonate, and other alkali/alkaline metal salts of physiologically acceptable acids such as acetates, glycinates, gluconates, borates, glycerophosphates or weak organic acids such as citric acid, phosphates, metal hydroxides such as sodium hydroxide and potassium hydroxide, and mixtures thereof. Examples of suitable pH adjusters are sodium bicarbonate and sodium carbonate, and mixtures thereof. It is preferable that the pH is higher at production of the compositions, such as a pH of 8 to 9, but the pH adjuster shall be capable of keeping the pH>6.5 throughout storage and consumption.

A preservative can be selected from selected from approved agents in food and pharmaceutical industry such as sorbic acid, sorbates, benzoic acid lactic acid and physiologically acceptable salts. A preferred preservative is potassium sorbate.

Taste or flavor enhancers include ammonium chloride, essential oils including distillations, solvent extractions or cold expressions of chopped flowers, leaves, peel or pulped whole fruit comprising mixtures of alcohols, esters, aldehydes and lactones or essences including either diluted solutions of essential oils or mixtures of synthetic chemical blends to match the desired flavour from for examples bergamot, eucalyptus, orange, mandarin, citrus, lemon, peppermint, mint, menthol, liquorice, wintergreen, tobacco, coffee, vanilla, lime, apple, peach and mixtures thereof. Further examples include artificial and natural flavours of brews and liquors, e.g. cognac, whiskey, rom, gin, sherry, port, and wine; eucalyptus, liquorice, and menthol.

Coloring agents can be selected from dyes containing chemical groups which absorb light including dyes, such as indigo carmine, amaranth, erythrosine, carbon black, titanium dioxide and any mixtures thereof.

Sweeteners can be natural sweeteners which are not fermentable in the mouth, or artificial sweeteners such as e.g. aspartame, acesulfame K, saccharin, cyclamates, Stevia extracts and other similar agents.

In aspects of the invention, the compositions are adapted to delivery to the oral cavity by contact with a mucous membrane. Such compositions comprise less than 50% of the matrix forming agent as defined above, preferably 0.1 to 10% (wt) more preferably 0.5 to 5% (wt) and comprise at least 30% (wt) of water, preferably 40 to 60% (wt) water. Such compositions can further comprise active agents, filling agents and excipients as defined above. In embodiments, the compositions comprise at least 40% (wt) of the filling agent that preferably comprises microcrystalline cellulose. Suitably, such compositions can be provided as conventional tobacco products, such as snus products with a defined amount of the composition is packaged in pouches. In one embodiment of such compositions, it comprises nicotine, β-glucan as the matrix forming agent, a filling agent comprising microcrystalline cellulose, at least 30% (w) water, a pH adjuster, an antioxidant and other excipients selected from one or more preservatives, taste/flavour enhancers and sweeteners, In one embodiment of such compositions, it comprises nicotine, β-glucan as the matrix forming agent, a filling agent comprising microcrystalline cellulose and optionally mannitol and/or other plant fibers, 40 to 60% (wt) water, a pH adjuster, an antioxidant and other excipients selected from one or more preservatives, taste/flavour enhancers and sweeteners. In one embodiment of such compositions, it comprises nicotine, a matrix forming agent comprising β-glucan and one or more other additional pharmaceutically/nutritionally acceptable gums as defined above, a filling agent comprising microcrystalline cellulose and optionally mannitol and/or other plant fibers, 40 to 60% (wt) water, a pH adjuster, an antioxidant and other excipients selected from one or more preservatives, taste/flavour enhancers and sweeteners.

In aspects of the invention, the compositions are adapted to delivery to the oral cavity as a lozenge or a tablet that gradually dissolves in contact with saliva. Such compositions can further comprise active agents, a filling agent and excipients as defined above. In certain embodiments, the filling agent can comprise a microcrystalline cellulose and In certain embodiments, the lozenge can have a coating comprising active agent, preferably comprising nicotine designed to provide the use with an initial quick does before the gradual release is established.

In other aspects of the invention, the compositions comprise more than 50% of the matrix forming agent and are configured as a film suitable for transmucosal delivery of active agent. The film compositions have a thickness of 0.01 to 7 mm, and optionally include a plasticizer. In an embodiment, these compositions comprise 0.05 to 20% (wt), preferably 5 to 10% (wt) of filling agent, preferably the filling agent is a microcrystalline cellulose and a plasticizer, preferably the plasticizer is selected from at least one of sorbitol and glycerol.

The components and the amount of the filling agent and the other named excipients may vary depending on the desired properties of the final product, for example to obtain attractiveness for oral use.

These and other embodiments will be more fully exemplified in the following detailed description.

In another general aspect, the present invention is directed to methods of producing the compositions for use in the oral cavity. The methods comprise dry mixing the filling agent and at least one of the matrix forming agent and an antioxidant; mixing the dry mixture with a first aqueous solution comprising a pH adjuster; adding a second aqueous solution comprising at least one of a preservative, a taste or flavour enhancer and a sweetener; adding a third aqueous solution comprising one or more biologically active agents and finally mixing all added components to a mixture with a suitable content of water.

In one embodiment of the method, the filling agent in the first step is dry mixed with the matrix forming agent and the antioxidant

In one embodiment of the method, the filling agent in the first step is dry mixed with the antioxidant and the third aqueous solution comprises the matrix forming agent and one or more biologically active agents.

The method can in another alternative be configured to produce a composition for use in the oral cavity by one or more further processing steps of the resulting mixture with at least one of filling in pouches, tablet forming or lozenge forming, extrusion, punching, casting, moulding, injection moulding, kneading, spinning, film, dilution to a sprayable dose form, forming and admixing with chewing gum base.

### Detailed and exemplifying description of the invention

Table 1 below further illustrates examples of oral compositions including suitable excipients.

**Table 1**

| **Ingredient** | **Use** | **Amount (wt %)** |
|---|---|---|
| Water | Humidification | 2-70% |
| Sodium Chloride | Taste | <15% |
| Microcrystalline cellulose | Filling agent | 5-95% |
| Sodium bicarbonate/ carbonate | pH adjuster | <2% |
| Beta-glucan >70% purity | Matrix forming agent | <5% |
| Ammonium chloride | Flavour | <2% |
| Potassium sorbate | Preservative | <0.2% |
| Xylitol | Sweetener | <5% |
| Acesulfame K/Stevia | Sweetener | <0.5% |
| Menthol/Spearmint/Lemon/Others | Flavour | <5% |
| Calcium ascorbate | Antioxidant | <5% |
| Nicotine | Active agent | <20% |

### Example 1

A specific example of a composition product made with the outlined methods is demonstrated in Table 2.

**Table 2**

| **Ingredient** | **Use** | **Amount (wt %)** |
|---|---|---|
| Water | Humidification | 45.93 |
| Microcrystalline cellulose | Filling agent | 42.10 |
| Sodium Chloride | Taste | 5.22 |
| Flavour | Smell and taste | 1.86 |
| Xylitol | Sweetener/Filling agent | 1.74 |
| Nicotine | Active agent | 0.89 |
| Beta-glucan of oat 98% purity from Xi'an Retalin Biotechnology, Xi'an, China | Matrix forming agent | 0.70 |
| Calcium ascorbate | Antioxidant | 0.70 |
| Ammonium chloride | Taste | 0.35 |
| Sodium bicarbonate | pH adjuster | 0.26 |
| Potassium sorbate | Preservative | 0.2 |
| Acesulfame K | Sweetener | 0.07 |

A composition of Table 2 without flavor, which may have a pH of about 8.5, suitable to be packaged in pouches as a snus type of product for use in the oral cavity was tested for stability of nicotine. Samples of 80 g of the composition of Table 2 and a commercial snus product based on tobacco were compared during 9 weeks at 40°C and 75% relative humidity (comparable to 10 months at 25°C without adjusted relative humidity).

**Table 3**

| | Composition of Table 2 | Commercial product |
|---|---|---|
| Water content % (wt) | 45.93 | 41.9 |
| Initial amount nicotine (mg) | 1.1 | 1.0 |
| Amount nicotine after 9 weeks (mg) | 1.1 | 0.74 |

Table 3 demonstrates that the beta-glucan and the antioxidant of the inventive compositions results in a significant increase in nicotine stability. A previous test with a composition similar to that of Table 2, but without any antioxidant demonstrates a comparable stability of nicotine to the commercial tobacco based product. This result indicates that a matrix forming agent comprising β-glucan according to the inventive compositions has a comparable capacity of preserving nicotine as the natural tobacco fibers. **In** conclusion, the combination of a matrix forming agent comprising β-glucan and a chelate binding antioxidant provides an effective long term stability of nicotine.

### Reference Example 2

**Table 4**

| **Ingredient** | **Use** | **Amount (wt %)** |
|---|---|---|
| Xylitol | Filling agent | 31.0 |
| Guar gum/Gum arabicum | Matrix forming agent | 31.0 |
| Maltodextrin | Matrix forming agent | 31.0 |
| Magnesium stearate | Lubricator | 1.0 |
| Nicotine | Active agent | 0.4 |
| Beta-glucan of oat 98% purity from Xi'an Retalin Biotechnology, Xi'an, China | Matrix forming agent | 0.7 |
| Acesulfam K | Sweetener | 0.3 |
| Sodium bicarbonate | pH adjuster | 0.4 |
| Anise oil | Aroma | 0.2 |
| Honey aroma/menthol/ mint | Aroma | 4 |

Table 4 shows an example of a lozenge or water soluble tablet comprising beta-glucan as a matrix forming agent.

The lozenge or tablet is made by dry mixing all components in Table 4, the resulting product is transferred to a conventional tablet forming machine and subjected to a high pressured and formed to tablets/lozenges. The tablets are spray coated and dries in coating pan to obtain a desirably tasting coating, comprising sweeteners, aroma and similar agents. The coating may optionally include nicotine to provide an initial dose.

### Example 3

Products according to the invention and Table 1 with 0.89 % (wt) nicotine, about 41 % (wt) water, 1 or 2 % of the beta-glucan as the matrix forming agent. The product was made with the two alternative methods outlined above. In Process 1, beta-glucan is dry mixed with the filling in the first step and a solution of nicotine is added in the third step. The product from Process 1 comprises 1% (wt) of beta-glucan. In Process 2, 1 or 2% (wt) of beta-glucan is added in the solution comprising nicotine in the third step. The product from Process 2 comprises 1 or 2% (wt) beta-glucan. The products were packaged in conventional snus pouches and benchmarked with two commercial tobacco free nicotine products, CP1 and CP2, respectively, comprising a microcrystalline cellulose as a filler, but not including any beta-glucan as a matrix forming agent. The product according to the invention, CP1 and CP2 were studied for stability and nicotine release. For the stability test, the products were all put in a heating cabinet at 40C and 75% humidity for 9 weeks (representing 7 months in room temperature.

**Table 5**

| | Initial Amount nicotine % (wt) | Amount nicotine after 7 months | % Nicotine loss |
|---|---|---|---|
| Product from Process 1 | 0.89 | 0.75 | 20 |
| CP1 | 1 | 0.74 | 26 |
| CP2 | 0.61 | 0.46 | 25 |

**Table 6**

| | Initial pH | pH after 7 months | % pH reduction |
|---|---|---|---|
| Product from Process 1 | 7.6 | 7.4 | 3 |
| CP1 | 8.4 | 7.9 | 6 |
| CP2 | 8.3 | 8 | 4 |

**Table 7**

| | Initial water content % (wt) | Water content % (wt) after 7 months | % change |
|---|---|---|---|
| Product from Process 1 | 41.2 | 43.9 | +7 |
| CP1 | 41.9 | 39.8 | -5 |
| CP2 | 45.3 | 47.9 | +6 |

The results of Tables 5 to 7 indicate that the product according to the invention comprising matrix forming agent with beta glucan stabilizes both nicotine and pH value during storage significantly better than either CP1 or CP2. The variations in water content between the product may result from different filling agents.

For testing the nicotine release capacity of compositions according to the invention, pouches with products were made as outlined above with Process 1 and Process 2 and compared with CP1. The products were orally tested by respondents, taken out after a defined time period and processed for remaining nicotine. A consumed pouch was chopped into a 100 ml glass bottle and exposed to ultrasound together with 5ml with Milli-Q water for 5 minutes. Thereafter, 100ml of 0.05M potassium hydroxide solution was added and the sample is shaken and then exposed to ultrasound for 60 minutes. The sample is shaken overnight on a vibrating table and is exposed to ultrasound an additional 30 minutes on the day after. Thereafter the sample is centrifuged and diluted to the desired level, the internal standard was added and the sample was then analyzed by LC / MS / MS. The averaged results of three tests are demonstrated in Table 8, below.

**Table 8**

| | Initial nicotine content (mg/g) | Nicotine content after 38 min (mg/g) | Release of nicotine (mg/g) | Nicotine bound to matrix forming agent |
|---|---|---|---|---|
| Product from process 1, 1%(wt) beta-glucan | 8.1 | 2.8 | 5.3 (65%) | 44% |
| Product from Process 2, 1%(wt) beta-glucan | 8.1 | 4.4 | 3.7 (45%) | 55% |
| Product from Process 2, 2%(wt) beta-glucan | 8.1 | 5.1 | 3.0 (37%) | 63% |
| CP1 | 8.9 | 5.7 | 3.2 (36%) | N/A |

Table 8 demonstrates that the amount of matrix forming agent of the inventive compositions can be used to control the release rate of nicotine. For example, doubling the amount of beta glucan from 1 to 2% (wt) generates 18% more nicotine bound to the matrix forming agent during a time period of 38 minutes. Also, the methods of manufacturing the compositions can be used to control the amount of bound nicotine to the beta-glucan of the matrix forming agent. The results of Table 8 demonstrate a higher amount of bound nicotine is obtained when nicotine is added together with the matrix forming agent as a third, final step in the manufacturing process.

## Claims

1. A composition for use in the oral cavity, said composition being adapted to delivery to the oral cavity by contact with a mucous membrane and comprising a biologically active agent, a matrix forming agent comprising β-glucan and a filling agent, wherein the composition has a pH of at least 6.5, and wherein the biologically active agent is nicotine; wherein the β-glucan comprises at least 30% β (1-3) β (1-4) glucan, and wherein the composition comprises less than 50 weight-% of the matrix forming agent and 30 weight-% to 60 weight-% of water.

2. A composition according to claim 1, wherein the β-glucan comprises 70 to 100% β (1-3) β (1-4) glucan, preferably the β-glucan is a cereal β-glucan, preferably obtained from oat.

3. A composition according to any previous claim, wherein the matrix forming agent comprises at least one additional pharmaceutically acceptable gum, preferably selected from alginate and salts thereof, xanthan, carrageenan, methyl cellulose, cudlan and pullulan, preferably the additional gum is a salt of alginate, more preferably sodium alginate.

4. A composition according to claim 2 or 3, wherein the matrix forming agent comprises at least 50 weight-% of cereal β-glucan.

5. A composition according to any one of the previous claims, comprising an antioxidant effective at a pH of at least 6.5, preferably effective at a pH of 8 to 9, preferably the antioxidant is a complex binding antioxidant, preferably selected from at least one of alkali and/or alkaline earth metal salts of ascorbate, calcium citrates, calcium lactates, calcium maleates, calcium tartrates, Ca-diNa-EDTA, calcium phosphates and ammonium citrates, more preferably the antioxidant is calcium ascorbate.

6. A composition according to any one of the previous claims, wherein the filling agent comprises a fiber material, preferably the fiber material is a plant fiber, more preferably the filling agent comprises natural or modified cellulose fibers and most preferably at least one microcrystalline cellulose.

7. A composition according to any one of the previous claims, wherein the filling agent comprises a polyol, preferably a polyol selected from one or more of mannitol, xylitol, sorbitol, maltitol and/or isomaltitol, lactitol and erythritol.

8. A composition according to any one of claims 1 to 7, comprising a filling agent comprising microcrystalline cellulose, preferably wherein the composition comprises 40 to 60 weight-% water.

9. A composition according to any one of the previous claims, further comprising at least one excipient selected from preservatives, taste or flavour enhancers, pH adjusters, plasticizers and sweeteners.

10. A method of manufacturing a composition according to claim 9, comprising:
(i) dry mixing the filling agent and the matrix forming agent comprising β-glucan and optionally an antioxidant;
(ii) mixing the dry mixture with a first aqueous solution comprising a pH adjuster;
(iii) adding a second aqueous solution comprising at least one of a preservative, a taste or flavour enhancer and a sweetener;
(iv) adding a third aqueous solution comprising nicotine and mixing all added components to a mixture with a suitable amount of water.

11. The method according to claim 10, comprising dry mixing the filling agent and the antioxidant and wherein the third aqueous solution comprises the matrix forming agent and the nicotine.

12. The method according to claim 10 or 11 configured to produce a composition for use in the oral cavity by one or more further processing methods of the resulting mixture with filling in pouches.

## Patentansprüche

1. Zusammensetzung zur Verwendung in der Mundhöhle, wobei die Zusammensetzung zur Abgabe in die Mundhöhle durch Kontakt mit einer Schleimhaut angepasst ist, und einen biologisch aktiven Wirkstoff, einen Matrixbildner, umfassend β-Glucan und einen Füllstoff, umfasst, wobei die Zusammensetzung einen pH-Wert von mindestens 6,5 aufweist, und wobei der biologisch aktive Wirkstoff Nicotin ist; wobei das β-Glucan mindestens 30 % β (1-3) β (1-4) Glucan umfasst, und wobei die Zusammensetzung weniger als 50 Gew.-% des Matrixbildners und 30 Gew.-% bis 60 Gew.-% Wasser umfasst.

2. Zusammensetzung nach Anspruch 1, wobei das β-Glucan 70 bis 100 % β (1-3) β (1-4) Glucan umfasst, bevorzugt ist das β-Glucan ein Getreide-β-Glucan, bevorzugt aus Hafer gewonnen.

3. Zusammensetzung nach einem vorstehenden Anspruch, wobei der Matrixbildner mindestens ein weiteres pharmazeutisch verträgliches Gummi umfasst, bevorzugt ist es aus Alginat und Salzen davon, Xanthan, Carrageen, Methylcellulose, Cudlan und Pullulan ausgewählt, bevorzugt ist das zusätzliche Gummi ein Salz von Alginat, bevorzugter Natriumalginat.

4. Zusammensetzung nach Anspruch 2 oder 3, wobei der Matrixbildner mindestens 50 Gew.-% Getreide-β-Glucan umfasst.

5. Zusammensetzung nach einem der vorstehenden Ansprüche, umfassend ein Antioxidans, das bei einem pH-Wert von mindestens 6,5 wirksam ist, bevorzugt ist es bei einem pH-Wert von 8 bis 9 wirksam, bevorzugt ist das Antioxidans ein komplexbindendes Antioxidans, bevorzugt ist es aus mindestens einem von Alkali- und/oder Erdalkalimetallsalzen von Ascorbat, Calciumcitraten, Calciumlactaten, Calciummaleaten, Calciumtartraten, Ca-diNa-EDTA, Calciumphosphaten und Ammoniumcitraten ausgewählt, bevorzugter ist das Antioxidans Calciumascorbat.

6. Zusammensetzung nach einem der vorstehenden Ansprüche, wobei der Füllstoff ein Fasermaterial umfasst, bevorzugt ist das Fasermaterial eine Pflanzenfaser, bevorzugter umfasst der Füllstoff natürliche oder modifizierte Cellulosefasern und besonders bevorzugt mindestens eine mikrokristalline Cellulose.

7. Zusammensetzung nach einem der vorstehenden Ansprüche, wobei der Füllstoff ein Polyol, bevorzugt ein Polyol, ausgewählt aus einem oder mehreren von Mannit, Xylit, Sorbit, Maltit und/oder Isomaltit, Lactit und Erythrit, umfasst.

8. Zusammensetzung nach einem der Ansprüche 1 bis 7, umfassend einen Füllstoff, umfassend mikrokristalline Cellulose, wobei die Zusammensetzung bevorzugt 40 bis 60 Gew.-% Wasser umfasst.

9. Zusammensetzung nach einem der vorstehenden Ansprüche, die weiter mindestens einen Hilfsstoff, ausgewählt aus Konservierungsmitteln, Geschmacks- oder Aromaverstärkern, pH-Einstellmitteln, Weichmachern und Süßstoffen, umfasst.

10. Verfahren zur Herstellung einer Zusammensetzung nach Anspruch 9, umfassend:
(i) Trockenmischen des Füllstoffs und des Matrixbildners, umfassend β-Glucan und wahlweise ein Antioxidans;
(ii) Mischen der Trockenmischung mit einer ersten wässrigen Lösung, umfassend ein pH-Einstellmittel;
(iii) Zugeben einer zweiten wässrigen Lösung, umfassend mindestens eines von einem Konservierungsmittel, einem Geschmacks- oder Aromaverstärker und einem Süßstoff;
(iv) Zugeben einer dritten wässrigen Lösung, umfassend Nikotin, und Mischen aller zugegebenen Komponenten zu einer Mischung mit einer geeigneten Menge Wasser.

11. Verfahren nach Anspruch 10, umfassend das Trockenmischen des Füllstoffs und des Antioxidans, und wobei die dritte wässrige Lösung den Matrixbildner und das Nikotin umfasst.

12. Verfahren nach Anspruch 10 oder 11, das zur Herstellung einer Zusammensetzung zur Verwendung in der Mundhöhle durch ein oder mehrere weitere Verarbeitungsverfahren der resultierenden Mischung mit Einfüllen in Portionsbeutel konfiguriert ist.

## Revendications

1. Composition pour une utilisation dans la cavité buccale, ladite composition étant adaptée à une délivrance dans la cavité buccale par contact avec une membrane muqueuse et comprenant un agent biologiquement actif, un agent de formation de matrice comprenant du β-glucane et un agent de remplissage, dans laquelle la composition présente un pH d'au moins 6,5, et dans laquelle l'agent biologiquement actif est la nicotine ; dans laquelle le β-glucane comprend au moins 30 % de β (1-3) β (1-4) glucane, et dans laquelle la composition comprend moins de 50 % en poids de l'agent de formation de matrice et de 30 % en poids à 60 % en poids d'eau.

2. Composition selon la revendication 1, dans laquelle le β-glucane comprend 70 à 100 % de β (1-3) β (1-4) glucane, de préférence le β-glucane est un β-glucane céréalier, de préférence obtenu à partir d'avoine.

3. Composition selon une quelconque revendication précédente, dans laquelle l'agent de formation de matrice comprend au moins une gomme pharmaceutiquement acceptable additionnelle, de préférence sélectionnée parmi l'alginate et des sels de celui-ci, le xanthane, la carraghénine, la méthylcellulose, le curdlane et le pullulane, de préférence la gomme additionnelle est un sel d'alginate, plus préférentiellement de l'alginate de sodium.

4. Composition selon la revendication 2 ou la revendication 3, dans laquelle l'agent de formation de matrice comprend au moins 50 % en poids de β-glucane céréalier.

5. Composition selon l'une quelconque des revendications précédentes, comprenant un antioxydant efficace à un pH d'au moins 6,5, de préférence efficace à un pH de 8 à 9, de préférence l'antioxydant est un antioxydant liant complexe, de préférence sélectionné parmi au moins un sel parmi des sels d'ascorbate de métaux alcalins et/ou alcalino-terreux, des citrates de calcium, des lactates de calcium, des maléates de calcium, des tartrates de calcium, Ca-diNa-EDTA, des phosphates de calcium et des citrates d'ammonium, plus préférentiellement l'antioxydant est l'ascorbate de calcium.

6. Composition selon l'une quelconque des revendications précédentes, dans laquelle l'agent de remplissage comprend un matériau fibreux, de préférence le matériau fibreux est une fibre végétale, plus préférentiellement l'agent de remplissage comprend des fibres de cellulose naturelles ou modifiées et le plus préférentiellement au moins une cellulose microcristalline.

7. Composition selon l'une quelconque des revendications précédentes, dans laquelle l'agent de remplissage comprend un polyol, de préférence un polyol sélectionné parmi un ou plusieurs polyols parmi le mannitol, le xylitol, le sorbitol, le maltitol et/ou l'isomaltitol, le lactitol et l'érythritol.

8. Composition selon l'une quelconque des revendications 1 à 7, comprenant un agent de remplissage comprenant de la cellulose microcristalline, de préférence dans laquelle la composition comprend de 40 % en poids à 60 % en poids d'eau.

9. Composition selon l'une quelconque des revendications précédentes, comprenant en outre au moins un excipient sélectionné parmi des conservateurs, des exhausteurs de goût ou d'arôme, des ajusteurs de pH, des plastifiants et des édulcorants.

10. Procédé de fabrication d'une composition selon la revendication 9, comprenant :
(i) mélanger à sec l'agent de remplissage et l'agent de formation de matrice comprenant du β-glucane et facultativement un antioxydant ;
(ii) mélanger le mélange sec avec une première solution aqueuse comprenant un ajusteur de pH ;
(iii) ajouter une deuxième solution aqueuse comprenant au moins un élément parmi un conservateur, un exhausteur de goût ou d'arôme et un édulcorant ;
(iv) ajouter une troisième solution aqueuse comprenant de la nicotine et mélanger tous les composants ajoutés en un mélange avec une quantité appropriée d'eau.

11. Procédé selon la revendication 10, comprenant le fait de mélanger à sec l'agent de remplissage et l'antioxydant et dans lequel la troisième solution aqueuse comprend l'agent de formation de matrice et la nicotine.

12. Procédé selon la revendication 10 ou la revendication 11 configuré pour produire une composition pour une utilisation dans la cavité buccale par une ou plusieurs méthodes de traitement supplémentaires du mélange résultant avec remplissage dans des sachets.
